(19)

Europäisches
Patentamt
European
Patent Office
Office européen
des brevets

(11) **EP 2 042 082 A1**

(12) **EUROPEAN PATENT APPLICATION**

(43) Date of publication:
**01.04.2009 Bulletin 2009/14**

(51) Int Cl.:
*A61B 5/00* (2006.01)    *G01N 21/64* (2006.01)

(21) Application number: **07117463.5**

(22) Date of filing: **28.09.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicants:
- **Stichting Voor De Technische Wetenschappen
  3527 JP Utrecht (NL)**
- **Erasmus University Medical Center Rotterdam
  3015 GE  Rotterdam (NL)**

(72) Inventors:
- **Amelink, Arjen
  2803 CB, Gouda (NL)**
- **Sterenborg, Henricus Josephus Cornelus Maria
  2901 RC, Capelle aan den IJssel (NL)**

(74) Representative: **Hatzmann, Martin et al
Vereenigde
Johan de Wittlaan 7
2517 JR Den Haag (NL)**

(54) **Method and apparatus for measuring fluorescence**

(57)    Fluorescence of fluorescent material in a medium is measured. Reflected light is received from the medium at the ends of a first and second optical fiber. Values of parameters of a model are determined that make intensities according to the model correspond to a difference between the detected intensities of the reflected light from the first and second optical fiber as a function of wavelength. Fluorescent light is received from the medium in response to light at a fluorescence exiting wavelength. A fluorescence signal of the medium is generated by normalizing the fluorescent intensity, or a signal derived therefrom, by an absorption factor obtained from the model with said values of the parameters and applied to the fluorescence exciting wavelength.

Fig.1

EP 2 042 082 A1

**Description**

Field of the invention

The invention relates to a method and apparatus for measuring fluorescence.

Background

The intensity of fluorescence can be used to measure the concentration of a substance in a turbid medium such as human or animal tissue. This may be applied for example when the substance is present in combination with other substances that do not fluoresce, but obstruct measurements of for example absorption spectra of the fluorescent substance.

Fluorescence is the emission of light by molecules of a substance at one or more characteristic wavelengths in response to irradiation with light at an excitation wavelength. The intensity of fluorescence at the location of the molecules is proportional to the concentration of the molecules, the intensity of the excitation at that location and molecular fluorescence efficiency. When the efficiency is known and the intensity of excitation can be calibrated the intensity of fluorescence can in principle be used to determine concentration of fluorescing substances in the presence of other components.

Unfortunately, actually measured fluorescence intensity can also be affected by other parameters of the medium such as absorption between the excitation light source and the location of the molecules and between that location and the detector of the fluorescent light. Scattering effects may also affect the measured fluorescence intensity. This complicates quantitative measurements of fluorescence, making it difficult to determine properties like concentration. The elimination of the dependence on absorption and scattering has proved to be an unpredictable art, among others because complex, variable light paths are involved. Only ad hoc models are available, and extensive experiments are needed to determine under what circumstances such models are reliable.

US patent No 6,377,842 describes how the dependence on absorption and scattering can largely be eliminated by using a very thin optical fiber. Unfortunately, use of a very thin optical fiber also reduces measurement sensitivity. The document addresses this problem by using a bundle of such fibers, each for supply the same excitation light and reception of the fluorescent response. The fibers are spaced in the bundle to avoid cross-coupling. This minimizes the relevant path length from the fiber to the molecules and back, and hence with much of the effect of absorption, but it requires a complex bundle. Moreover, the effect of absorption is merely reduced rather than accounted for. For large absorption coefficients no quantitative results are possible unless absorption dependence of the fluorescence measurement is accounted for.

In an alternative approach the ratio between the fluorescent light and an additional measurement of reflection is computed to eliminate the effect of absorption. This approach relies on specific assumptions about reflection, absorption and fluorescence. This approach is exemplified by an article titled: "Monitoring photosensitizer concentration by use of a fiber-optic probe with a small source-detector separation" by Murat Canpolat and Judith R. Mourant and published in APPLIED OPTICS y Vol. 39, No. 34 y 1 December 2000, pages 6508-6514. Herein separate fibers are used to supply excitation light and to receive reflected and fluorescent light, at the excitation wavelength and other wavelengths respectively. From this the concentration of fluorescent molecules can be measured under the assumption that absorption is proportional to fluorescence plus a known background absorption and that the light paths involved in reflection and absorption are the same. The concentration is determined by dividing the measured fluorescence intensity by the measured reflectance intensity and the result of a calibration measurement performed with a medium with known absorption. Unfortunately, the assumptions underlying this approach are not always met under practical circumstances. Moreover, their fluorescence over reflectance ratio varies rather strongly with scattering, which strongly limits their technique.

Use reflection to remove the effects of absorption is also known from an article titled "Turbidity-free fluorescence spectroscopy of biological tissue", by Qingguo Zhang, Markus G. Müller, Jun Wu, and Michael S. Feld in OPTICS LETTERS October 1, 2000, Vol. 25, No. 19, pages 1451-1453. Here a complex series of measurements is needed to correct for the effect of absorption.

Summary

Among others, it is an object to provide for a measurement of fluorescence of material in a medium that is less dependent on absorption and assumptions about the medium.

A method of measuring fluorescence of fluorescent material in a medium according to claim 1 is provided. Herein differential reflection measurements, obtained for a range of wavelengths are used to compute a correction of fluorescence measurements. It has been found that use of the differential reflection measurement over the range of wavelengths leads to a reliable correction for the effects of absorption in fluorescence measurements, also when there is heavy absorption at the fluorescence exciting wavelength.

In an embodiment the same optical fibers may be used to perform the reflection and fluorescence measurements. This makes a probe for performing the measurements more compact.

Brief description of the drawing

These and other advantageous aspects will become apparent from a description of exemplary embodiments, using the following figures

Figure 1     shows a fluorescence measurement apparatus
Figure 2     shows a flow chart of operation
Figure 3     shows graphs of absorption coefficients

Detailed description of exemplary embodiments

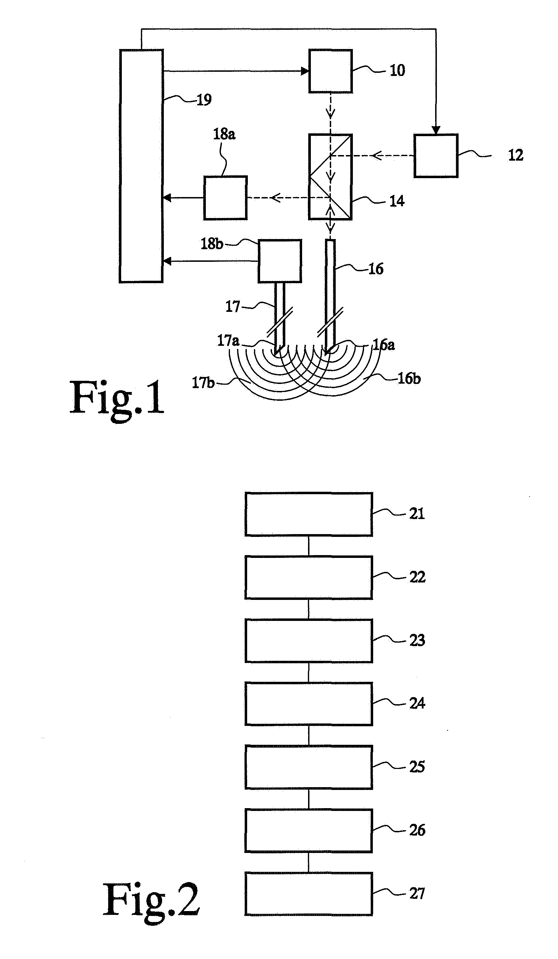

Figure 1 shows a fluorescence measurement apparatus. The apparatus comprises a first and second light source 10, 12, a coupler 14, a first optical fiber 16, a second optical fiber 17, a first detector 18a a second detector 18b and a data processing device 19. First optical fiber 16 as an output end 16a located adjacent an input/output end 17a of second optical fiber 17. The output end 16a and the input end 17a are oriented in parallel, so that a first region 16b that is illuminated from first optical fiber 16 substantially overlaps in the far field with a second region 16b from which second optical fiber 17 receives light. In an embodiment, fibers 16, 17 each have a diameter of 0.4 millimeter, and the central axes of the fibers are spaced at slightly more than 0.4 millimeter from each other. When the ends 16a, 17a of optical fibers 16, 17 are directed at a medium that scatters light, regions 16b, 17b typically have a rounded shape as shown, due to scattering. However, it should be noted that the shapes of successively less intensely illuminated regions are shown only symbolically. The regions may have different shapes. For example, they may depend in a different way on the boundary of the medium and they may extend further from the ends 16a, 17a of the fibers 16, 17.

In operation, the medium may contain variable amounts of fluorescent substances, such as photosensitizers, and other substances, such as hemoglobin, that contribute in a major way to absorption of the medium but do not fluoresce, or fluoresce in a distinguishable way. It is desirable to obtain measurements of the fluorescence wherein the effect of absorption has been removed.

Coupler 14 is configured to transmit the light from first and second light source 10, 12 to first optical fiber 16, and to direct light that is received back in first optical fiber 16 to first detector 18a. Second optical fiber 17 directs received light to second detector 18b. First and second detector 18a,b have outputs coupled to data processing device 19. In addition a third detector (not shown) coupled to data processing device 19 may be provided for measuring intensity of light that is supplied to first optical fiber 16. However, at least if the output intensity of light sources 10, 12 is constant and known such a third detector may be omitted.

Figure 2 shows a flow chart of operation. During operation, the input/output end 16a and the input end 17a of the optical fibers 16, 17 are directed at a medium that contains fluorescent material. In a first step 21, data processing device 19 activates first light source 10, which is a broadband light source, e.g. a white light source, or a light source containing light with intensity at wavelengths between at least 350 and 850 nanometer. Alternatively a plurality of narrowband light sources may be used, which are applied simultaneously, or the wavelength of an adjustable narrowband light source may be swept. In a second step 22 data processing device 19 reads intensity I2a(wl), I2b(wl) measurements as a function of wavelength wl from first and second detector 18a,b while first light source 10 is on. The measured intensities I2a(wl), I2b(wl) are a result of supply of the light from first light source 10 to the medium at via first optical fiber 16 and reception back from the medium via optical fibers 16, 17, respectively.

In a third step 23 data processing device 19 deactivates first light source 10 and activates second light source 12, which is a narrow band light source, e.g. a laser, which emits light of a wavelength w10 that will cause fluorescence by the fluorescent material in the medium. A wavelength of 405 nanometer may be used for example.

In a fourth step 24 data processing device 19 reads intensity measurements I4a(wl), I4b(wl) as a function of wavelength from first and second detector 18a, 18b while second light source 12 is on, and while optical fibers 16, 17 are directed at the same medium as in second step 22 within a time interval from the measurement of second step 22 that is short enough to ensure that the optical properties of the medium have not changed in a significant way. In this sense the second and fourth step are executed substantially simultaneously. Intensity may be measured over a range of wavelengths extending from 500 to 800 nanometer, for example. The measured intensities I4a(wl) and I4b(wl) in fourth step 24 are a result of supply of the light from second light source 12 to the medium at via first optical fiber 16 and reception back from the medium via optical fibers 16 and 17.

In a fifth step 25 data processing device 19 processes the data representing intensities I2a(wl), I2b(wl), that were obtained in second step 22. If desired, the intensities measured by the detectors may be calibrated relative to each other, to obtain comparable data representing intensity in the medium at the ends 16a, 17a of the fibers 16, 17, eliminating the effect of differences in collection efficiency, transmission etc. The intensities measured by second detector 18b are subtracted from the intensities measured by second detector 18a for respective wavelengths. As will be appreciated, this means that in the resulting difference D2(wl)=I2a(wl)-I2b(wl) the contribution of light from the overlapping part of regions 16b, 17b is removed from the intensity measured by first detector 18a. Only the contribution from the non-overlapping part of the region 16b mostly near the input/output end 16a of first optical fiber 16 is retained in the difference D2(wl). The difference D2 is normalized by dividing with the difference Dwhite- Dblack between intensity obtained with perfect reflection and perfect absorption.

This is described in an article titled "Measurement of the local optical properties of turbid media by differential path-length spectroscopy", by Amelink et al, and published in APPLIED OPTICS Vol. 43, No. 15, 20 May 2004, page 3048-3054. In this way a normalized intensity difference D2n=D2/(Dwhite-Dblack) is obtained. If desired the intensities may also be compensated for intensities due to geometrical aspects of the fibers, which can be obtained from measurements in reference media. If desired the intensities may also be compensated for measured intensity variations of the light source 10.

Moreover, the data processing in fifth step 25 comprises fitting parameters of a model for reflection from the medium so that the model optimally matches the measured reflection as a function of wavelength. The model may take the form of a formula for reflection as a function of wavelength, with a number of parameters in the formula, whose values may be selected to realize an optimal fit. An example of such a formula, for use when the medium is tissue with blood vessels, has been published in the cited article by Amelink and in an article titled "In vivo measurement of the local optical properties of tissue by use of differential path-length spectroscopy", by Amelink et al. in OPTICS LETTERS May 15, 2004 , Vol. 29, No. 10, pages 1087-1089. This formula has the form

$$D2n(wl)=C* wl^{-b} \exp\{ -0.8*VD*ma1(wl)*[S*ma2(wl) + (1-S)*ma3(wl) ] \}$$

Herein D2 is the normalized reflection intensity as a function of wavelength wl, C is a wavelength independent factor, b is an exponent that depends on the size of scattering particles. VD is a factor that may depend on fiber diameter and microvascular blood volume fraction, S is the microvascular blood oxygenation.

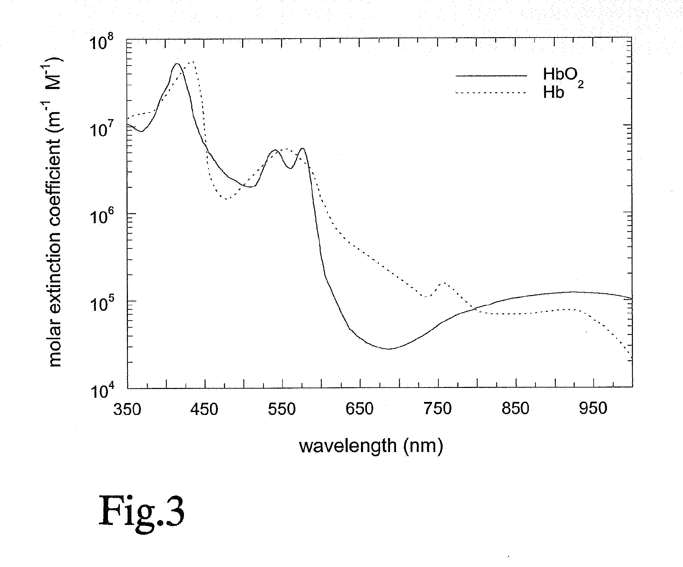

Figure 3 show the wavelength dependence of ma2 and ma3 (termed extinction coefficient in the figure) normalized for a concentration of one mole, which are the absorption coefficients of fully oxygenated whole blood and fully de-oxygenated whole blood respectively.

The function ma1 is an absorption factor that accounts for the inhomogeneous distribution of blood in the tissue. A wavelength dependence of this factor as a function of average blood vessel diameter D is known, e.g. from the cited article by Amelink in Optics Letters.

The model has the factor C, the exponent b, the factor VD, blood oxygenation S and the vessel diameter D as parameters. When the reflection intensity R is measured at a sufficient number of wavelengths, all of these parameters can be estimated from the wavelength dependence of the measured reflection intensity. Preferably measurements at more than a minimally sufficient number of wavelengths are used. In this case parameters may be selected that minimize a measure of deviation between the measured values and their predictions from the model, such as a sum of squares of the deviation for individual wavelengths. It may be possible to extract the parameters from D2(wl) without normalization, if the normalization factor can be taken as part of the factor C.

In a sixth step 26, data processing device 19 processes the data representing I4a(wl) and/or I4b(wl) that was obtained in fourth step 24. At wavelengths other than the excitation wavelength this data represents intensity that is proportional the fluorescence intensity as a function of wavelength. Data processing device 19 subtracts first intensities I4b(wl) measured by second detector 18b from the intensities I4a(wl) measured by second detector 18a for respective wavelengths sixth step 26, optionally after relative calibration of the detectors 18a,b. As will be appreciated, this means that the contribution of light from the overlapping part of regions 16b, 17b has been removed from the difference D4(wl) =I4a(wl)-I4b(wl).

If desired the intensities may be compensated intensities due to geometrical aspects of the fibers and/or for measured intensity of the second light source 12. The resulting intensities D4(wl) from sixth step 26 at wavelengths wl other than the excitation wavelength are proportional to fluorescence mostly near the input/output end 16a of first optical fiber 16, but they also depend on light absorption. In the case of the differential intensity D4(wl) fluorescence in the non-overlapping part of the region 16b contributes.

In a seventh step 27 data processing device 19 processes the data D4(wl) to remove effects of absorption according to the formula

$$F(wl) = D4(wl)/ \exp\{ -0.8*VD*ma1(wl0)*[S*ma2(wl0) + (1-S)*ma3(wl0) ] \}$$

The second factor is derived from the reflection measurements at a plurality of wavelengths via the result of fifth step 25 and extrapolated to the wavelength w10 of the light from first optical fiber 16 that is used to excite fluorescence. As can be seen the known wavelength dependence of ma1(wl), ma2(wl) and ma3(wl) and the know wavelength dependence of intrinsic reflection (the exponential dependence $wl^{-b}$ in the case of the formula for reflection) make it possible to isolate this factor.

Although the formula shows multiplication by an exponent obtained from the reflection measurements, it should be realized that in practice this exponent can be estimated in several ways. In one embodiment it may be derived using the parameters VD and S. In an alternative embodiment it may be derived by interpolating $D2n(wl)$ to w10 and dividing by extracted values of $C^* w10^{-b}$. many computational approaches may be followed that have approximately the same result.

The corrected fluorescence F(wl) represents fluorescence intensity substantially without dependence on absorption in the medium. Experiments in media with known fluorescence properties have shown this independence. It may be noted that, surprisingly, use of the whole exponential factor from the reflection measurement yields the best elimination of dependence on absorption. In the reflection measurements this corresponds to the optical path from the end 16a of first fiber 16 to the point in the medium where reflection occurs and back to the first or second optical fiber 16, 17 after reflection. At first sight one might expect that only the absorption in the first part of this optical path is relevant to fluorescence, as the return light back to the fibers is at a fluorescent wavelength wl at which much less absorption occurs than at the excitation wavelength wl0. Hence, one might expect that only a fraction of the term in the exponent for reflection should be used. Contrary to this, it has been found that use of the whole exponent for the entire path in the refection measurement yields the best elimination of the dependence on absorption of the fluorescence measurement.

The resulting fluorescence intensity F(wl) is proportional to the concentration of fluorescent material in the medium and fluorescence efficiency of the material as a function of wavelength. By means of a calibration measurement the dependence on geometrical factors can be eliminated. The resulting fluorescence intensity F(wl) may be used for example in a fluorescence measuring apparatus, or as part of a system to control other equipment in the system. Thus, for example, processing device 19 may be coupled for example to a treatment light source (not shown) for controlling a treatment intensity dependent on fluorescence intensity, or to an oxygen supply unit (unit) for use in controlling the oxygen concentration of air that is supplied to a patient dependent on fluorescence intensity. When used in a fluorescent measuring apparatus data processing device 19 may write the resulting intensity value to a display device (not shown), or use it to control display of a graph of fluorescence at a display device (not shown), or data processing device 19 may write it to a memory device (not shown) for use by another device.

Although an embodiment has been described wherein fluorescence is measured in tissue with blood vessels, it should be appreciated that a similar technique may be applied to other media with fluorescent material, in which case other models may be needed to extract parameters from reflection measurements. Thus, for example when a mixture of other materials is used, in the fifth step 25 the term

$$S*ma2(wl) + (1-S)*ma3(wl)$$

may be replaced in the model with a sum of terms containing products of the concentration of the respective materials and their wavelength dependent absorption coefficients, dependent on the materials that are known to be possibly present in the medium. Similarly, the term ma1(wl) may be replaced by one or by another factor that accounts for the inhomogeneous distribution of material in the medium.

Furthermore, in fifth step 25, various reflection models may be used, dependent on the materials that are used. The exponent term $wl^{-b}$, which corresponds to Mie scattering, is used based on the discovery that reflection in tissue with blood vessels can be described with sufficient accuracy by Mie scattering. In other media Rayleigh scattering may be dominant, in which case the exponent term $wl^{-b}$ may be replaced by $wl^{-4}$. If the relative importance of Mie and Rayleigh scattering is not known the exponent term $wl^{-b}$ may be replaced by a term $a1*wl^{-b}+a2*wl^{-4}$, wherein a1 and a2 are additional parameters that may be fitted to the measurement in fifth step 25. As will be appreciated this will have a similar result as using only the term $wl^{-b}$ if no significant Rayleigh scattering occurs. Any other known function with or without a limited number of free parameters may be used instead of the exponent term $wl^{-b}$, dependent on the properties of the particular medium in which fluorescence is measured.

The use of differential measurements of reflection, including subtraction of I2b(wl) from I2a(wl) has the advantage that it is avoided that a much larger region in the medium is used for the reflection measurement than for the fluorescence wavelength. In materials with efficient fluorescence the region of fluorescence is usually quite small due to absorption. As the reflection measurement involves a plurality of wavelengths, which may include wavelengths at which there is little absorption, the region where reflection occurs may be larger. This difference may affect the accuracy of elimination of the effect of absorption from fluorescence. By using differential measurements this may be avoided. Although an embodiment has been shown wherein the light for reflection is supplied via the first optical fiber, it should be realized that a third optical fiber (possibly with a much wider diameter than the first and second optical fiber) may be used to supply this light, as differential measurement with the first and second optical fiber will also reduce the contributing region in this case.

The use of differential measurement for fluorescence, including subtraction of I4b(wl) from I4a(wl) may be avoided by using a measurement of only I4a(wl) or even I4b(wl) instead of D4(wl). The region in the medium that contributes to the measured quantity is small in any case when absorption is high. Use of the differential D4(wl) in combination with the measured D2(wl) has the advantage that the regions that contribute to the measured reflection and fluorescence are more similar, which may increase accuracy of the removal of absorption dependence.

Although an embodiment is shown wherein the same fibers are used for fluorescence and reflection measurements, it should be appreciated that in practice different fibers, directed at comparable media, may be used. Use of the same fibers has the advantage of compactness and better comparable measurements, which may increase accuracy of the removal of absorption dependence. Although an embodiment is shown wherein first fiber 16 is used to supply light both for fluorescence measurement and reflection measurement, alternatively second fiber 17 may be used to supply light for the fluorescent measurement, with a corresponding modification of coupler. Although an embodiment has been shown wherein the light source for reflection and the light source for exciting fluorescence are on only when the other is off during measurment, it should be realized that alternatively both may be on simultaneously during measurement when reflection can be measured at wavelengths that are not involved in fluorescence measurement. However, it is preferred to measure with only one of the light sources on, to minimize the risk of cross-talk.


**Claims**

1.   A method of measuring fluorescence of fluorescent material in a medium, the method comprising

   - supplying first light to the medium at a plurality of wavelengths;
   - receiving reflected light from the medium in response to the first light at the ends of a first and second optical fiber respectively, the ends of the first and second optical fiber being located adjacent one another;
   - detecting reflected intensities of the reflected light received from the first and second optical fiber at said plurality of wavelengths;
   - providing a model of reflection;
   - computing values of parameters of the model that make intensities according to the model correspond to a difference between the detected intensities of the reflected light from the first and second optical fiber as a function of wavelength;
   - supplying second light to the medium at a fluorescence exciting wavelength;
   - receiving fluorescent light from the medium in response to the second light via one of the first optical fiber, the second optical fiber or a third optical fiber;
   - detecting a fluorescent intensity at a fluorescent response wavelength of the fluorescent light received via said one of the first optical fiber, the second optical fiber or a third optical fiber;
   - computing a fluorescence signal of the medium by normalizing the fluorescent intensity, or a signal derived therefrom, by an absorption factor obtained from the model with said values of the parameters and applied to the fluorescence exciting wavelength.

2.   A method according to claim 1, wherein the signal derived from the fluorescent intensity is derived by

   - receiving the fluorescent light via both the first and second optical fiber;
   - detecting fluorescent intensities received via the first and second optical fiber at said fluorescent response wavelength;
   - subtracting the detected fluorescent intensities.

3.   A method according to any one of the preceding claims, wherein said model of reflection comprises an absorption term that is a sum of products of concentrations of respective substances that are potentially present in the medium and absorption coefficients as a function of wavelengths of said respective substances, the parameters of which the values are computed comprising the concentrations.

4. A method according to claim 3, wherein the respective substances include oxygenated blood and de-oxygenated blood.

5. A method according to claim 3 or 4, wherein the model represents reflection R as a function of wavelength wl as a formula

$$R(wl)=C* \ wl^{-b} \ exp\{ \ -f*P \ \}$$

wherein P is said sum and f, C and b are parameters of the model.

6. A method according to claim 3 or 4, wherein the model represents reflection R as a function of wavelength wl as a formula

$$R(wl)=C* \ (a1*wl^{-b} +a2*wl^{-4})* \ exp\{ \ -f*P \ \}$$

wherein P is said sum and a1, a2, f, C and b are parameters of the model.

7. A method according to claim 3, wherein the model represents reflection R as a function of wavelength wl as a formula

$$R(wl)=C* \ wl^{-b} \ exp\{ \ -f*M(wl)*P \ \}$$

wherein P is said sum and f, C and b are parameters of the model and M(wl) is an absorption factor that accounts for the inhomogeneous distribution of blood in the medium and is dependent on average blood vessel diameter in the medium, said average blood vessel diameter being a parameter of the model.

8. A method according to any one of the preceding claims, wherein the absorption factor that is used to normalize the fluorescent intensity, or the signal derived therefrom, represents absorption in paths from said one of the first, second and third optical fiber to locations in the medium where reflection occurs and back to the first and second optical fiber from these locations.

9. An apparatus for measuring fluorescence of fluorescent material in a medium, the apparatus comprising

- a light source arrangement for supplying first light over a range of wavelengths and second light at a fluorescence exciting wavelength;
- a first and second optical fiber having ends adjacent one another for receiving light from the medium;
- a first and second detector coupled to the first and second optical fiber respectively, for detecting a reflection intensity of reflected light entering the first and second optical fiber from said ends respectively, as a function of wavelength after reflection of the first light by the medium;
- one of a third optical fiber, the first optical fiber and the second optical fiber;
- one of a third detector coupled to the third optical fiber, the first detector and the second detector, for detecting fluorescent intensity at a fluorescent response wavelength of fluorescent light received from the medium via said one of the first, second and third optical fiber in response to the second light;
- a data processing circuit coupled to the first detector, the second detector and the third detector, if present, and configured to compute values of parameters of a reflection model that make reflection intensities according to the reflection model correspond to a difference between the detected intensities of the reflected light from the first and second optical fiber as a function of wavelength and to normalize the fluorescence intensity, or a signal derived therefrom, by an absorption factor obtained from the model with said values of the parameters and applied to the fluorescence exciting wavelength.

7

# Fig.1

# Fig.2

Fig.3

**European Patent Office**

**EUROPEAN SEARCH REPORT**

Application Number

EP 07 11 7463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/057757 A (MASSACHUSETTS INSTITUE OF TECH [US]; GEORGAKOUDI IRENE [US]; FELD MICH) 25 July 2002 (2002-07-25) | 1,2,9 | INV. A61B5/00 G01N21/64 |
| Y | * abstract * <br> * figures 1,16 * <br> * page 2, line 26 - page 3, line 31 * <br> * page 4, line 7 - line 10 * <br> * page 7, line 24 - line 25 * <br> * page 21, line 24 - page 22, line 4 * <br> * page 23, line 10 - line 20 * <br> * page 24, line 3 - line 11 * <br> ----- | 3-8 | |
| X | WO 99/57529 A (UNIV TEXAS [US]; UTZINGER URS [US]; DURKIN ANTHONY [US]; FUCHS HOLGER) 11 November 1999 (1999-11-11) <br> * abstract * <br> * figure 1 * <br> * page 5, line 1 - line 9 * <br> * page 85, line 2 - line 9 * <br> * claims 1-8,30 * <br> ----- | 1,9 | |
| Y,D | AMELINK, A AND STERENBORG, H.J.C.M.: "In vivo measurement of the local optical properties of tissue by use of differential path-length spectroscopy" OPTICIS LETTERS, vol. 29, no. 10, 15 May 2004 (2004-05-15), pages 1087-1089, XP002470841 * the whole document * <br> ----- | 3-8 | TECHNICAL FIELDS SEARCHED (IPC) <br> A61B <br> G01N |
| A | US 5 452 723 A (WU JUN [US] ET AL) 26 September 1995 (1995-09-26) * abstract * <br> ----- | 1 | |
| A | US 4 768 513 A (AGENCY IND SCIENCE TECHN) 6 September 1988 (1988-09-06) * abstract * * figure 4 * <br> ----- | 1 | |

The present search report has been drawn up for all claims

| Place of search | Date of completion of the search | Examiner |
|---|---|---|
| Munich | 3 April 2008 | Tommaseo, Giovanni |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   .......................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 07 11 7463

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2008

| Patent document cited in search report | | Publication date | Patent family member(s) | | Publication date |
|---|---|---|---|---|---|
| WO 02057757 | A | 25-07-2002 | CA | 2431769 A1 | 25-07-2002 |
| | | | EP | 1352231 A2 | 15-10-2003 |
| | | | JP | 2004518124 T | 17-06-2004 |
| | | | US | 2003013973 A1 | 16-01-2003 |
| | | | US | 2002143243 A1 | 03-10-2002 |
| WO 9957529 | A | 11-11-1999 | AU | 3970799 A | 23-11-1999 |
| US 5452723 | A | 26-09-1995 | NONE | | |
| US 4768513 | A | 06-09-1988 | JP | 62247232 A | 28-10-1987 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 6377842 B

**Non-patent literature cited in the description**

• *APPLIED OPTICS,* 01 December 2000, vol. 39 (34), 6508-6514
• **QINGGUO ZHANG ; MARKUS G. MÜLLER ; JUN WU ; MICHAEL S.** Turbidity-free fluorescence spectroscopy of biological tissue. *Feld in OPTICS LETTERS,* 01 October 2000, vol. 25 (19), 1451-1453

• Measurement of the local optical properties of turbid media by differential path-length spectroscopy. *APPLIED OPTICS,* 20 May 2004, vol. 43 (15), 3048-3054
• **AMELINK et al.** In vivo measurement of the local optical properties of tissue by use of differential path-length spectroscopy. *OPTICS LETTERS,* 15 May 2004, vol. 29 (10), 1087-1089
• **AMELINK.** *Optics Letters*